(19) **Europäisches Patentamt**

**European Patent Office**

**Office européen des brevets**

(11) **EP 1 083 867 B1**

(12) **FASCICULE DE BREVET EUROPEEN**

(45) Date de publication et mention
de la délivrance du brevet:
**20.09.2006 Bulletin 2006/38**

(21) Numéro de dépôt: **99923692.0**

(22) Date de dépôt: **08.06.1999**

(51) Int Cl.:
*A61K 8/81* (2006.01)　　　*A61K 8/85* (2006.01)
*A61K 8/899* (2006.01)　　　*A61Q 5/06* (2006.01)

(86) Numéro de dépôt international:
**PCT/FR1999/001347**

(87) Numéro de publication internationale:
**WO 1999/063955 (16.12.1999 Gazette 1999/50)**

(54) **COMPOSITION COSMETIQUE COMPRENANT AU MOINS UN POLYMERE COLLANT DU TYPE POLYESTER RAMIFIÉ SULFONÉ ET AU MOINS UN POLYMERE FIXANT**

KOSMETISCHE ZUSAMMENSETZUNG, DIE MINDESTENS EIN KLEBRIGES POLYMER VOM TYP DER SULFONIERTEN VERZWEIGTEN POLYESTER UND MINDESTENS EIN FIXIERENDES POLYMER ENTHÄLT

COSMETIC COMPOSITION COMPRISING AT LEAST A TACKY BRANCHED SULFONATED POLYESTER POLYMER AND AT LEAST A FIXING POLYMER

(84) Etats contractants désignés:
**DE ES FR GB IT**

(30) Priorité: **11.06.1998 FR 9807376**

(43) Date de publication de la demande:
**21.03.2001 Bulletin 2001/12**

(60) Demande divisionnaire:
**05291945.3 / 1 642 564**

(73) Titulaire: **L'ORÉAL**
**75008 Paris (FR)**

(72) Inventeurs:
- **ROLLAT-CORVOL, Isabelle**
  **F-92100 Boulogne-Billancourt (FR)**
- **SAMAIN, Henri**
  **F-91570 Bièvres (FR)**

(74) Mandataire: **Bourdeau, Françoise et al**
**L'OREAL - D.I.P.I.**
**25-29 Quai Aulagnier**
**92600 Asnières (FR)**

(56) Documents cités:
**EP-A- 0 524 346**　　　**EP-A- 0 551 749**
**WO-A-95/00105**　　　**WO-A-95/33437**
**US-A- 5 266 303**　　　**US-A- 5 441 728**

**Description**

L'invention a pour objet une composition cosmétique pour les fibres kératiniques telles que les cheveux comprenant, dans un milieu cosmétiquement acceptable, au moins un polymère collant de température de transition vitreuse inférieure à 20°C et au moins un polymère fixant de température de transition vitreuse supérieure à 15°C, le polymère collant étant un polyester sulfonique ramifié. Elle vise également un procédé de traitement des fibres kératiniques telles que les cheveux, en particulier un procédé de fixation et/ou de maintien de la coiffure, mettant en œuvre ladite composition ainsi que l'utilisation de cette composition dans ou pour la fabrication d'une formulation cosmétique de coiffage.

Au sens de la présente invention, on entend par "fibres kératiniques", les cheveux, les cils et les sourcils et par "polymère collant", un polymère qui, après application par pression sur un polymère identique, résiste à une tentative de séparation.

La fixation de la coiffure est un élément important du coiffage qui consiste à maintenir la mise en forme déjà réalisée ou à mettre en forme les cheveux et à les fixer simultanément. On entend par "polymère fixant", un polymère qui maintient en forme les cheveux ou qui permet de mettre en forme les cheveux et de les fixer simultanément.

Les produits capillaires pour la mise en forme et/ou le maintien de la coiffure les plus répandus sur le marché de la cosmétique sont des compositions à pulvériser essentiellement constituées d'une solution le plus souvent alcoolique ou aqueuse et d'un ou plusieurs matériaux, généralement des résines polymères, dont la fonction est de former des soudures entre les cheveux, appelés encore matériaux fixants, en mélange avec divers adjuvants cosmétiques. Cette solution est généralement conditionnée soit dans un récipient aérosol approprié mis sous pression à l'aide d'un propulseur, soit dans un flacon pompe.

On connaît également les gels ou les mousses de coiffage qui sont généralement appliqués sur les cheveux mouillés avant de faire un brushing ou une mise en plis. A la différence des laques aérosols classiques, ces compositions présentent l'inconvénient de ne pas permettre la fixation des cheveux dans une forme déjà réalisée. En effet, ces compositions sont essentiellement aqueuses et leur application mouille les cheveux et ne peut donc maintenir la forme initiale de la coiffure. Pour mettre en forme et fixer la coiffure, on doit donc ensuite effectuer un brushing ou un séchage.

La plupart des compositions de l'état de la technique présentent le même inconvénient de ne pas fixer ou maintenir la coiffure suffisamment durablement. Ainsi, la forme donnée initialement à la coiffure s'estompe progressivement au cours de la journée, et ceci d'autant plus vite d'ailleurs que la personne est en mouvement. En conséquence, il est souvent nécessaire de recommencer l'ensemble des opérations de coiffage et de fixation si l'on souhaite retrouver la coiffure initiale. Le document US 5 266 303 décrit une formulation aérosol capillaire comprenant un polyester sulfonique linéaire ayant une température de transition vitreuse d'environ 36 à environ 40°C avec un polyvinyllactame.

On recherche donc des compositions de coiffage qui procurent un effet de fixation et de maintien suffisamment forts pour que la coiffure résiste convenablement dans le temps aux diverses sollicitations.

Enfin, les compositions destinées à la fixation de la coiffure présentent parfois l'inconvénient d'altérer les propriétés cosmétiques des cheveux. Ainsi, les cheveux peuvent devenir rêches, difficiles à démêler, perdre leur toucher et leur aspect agréables. On recherche donc des compositions coiffantes procurant de bonnes propriétés cosmétiques, notamment en terme de démêlage, de douceur et de toucher.

Il existe donc un besoin de trouver des compositions cosmétiques, notamment pour le coiffage, qui ne présentent pas l'ensemble des inconvénients indiqués ci-dessus.

De manière surprenante et inattendue, la Demanderesse a découvert que lorsque l'on associe des polymères collants polyesters sulfoniques ramifiés avec certains polymères fixants, il est possible d'obtenir des compositions cosmétiques répondant aux exigences exprimées ci-dessus.

L'invention a donc pour objet une composition cosmétique pour les fibres kératiniques telles que les cheveux comprenant, dans un milieu cosmétiquement acceptable, au moins un polymère collant de température de transition vitreuse (Tg) inférieure à 20°C et au moins un polymère fixant de température de transition vitreuse(Tg) supérieure à 15°C, le polymère collant étant un polyester sulfonique ramifié.

De manière avantageuse, on choisit un polymère collant présentant un profil de décollement défini par au moins une force maximale de décollement $F_{max}$ > 3 Newton, et de préférence supérieure à 5N.

Plus avantageusement encore, le profil de décollement est défini en outre par une énergie de séparation $E_{s(M/V)}$ du matériau mis en contact avec une surface en verre, inférieure à 300 $\mu$J, lorsque la température de transition vitreuse du polymère collant est inférieure à -15°C.

La force maximale de décollement Fmax est la force maximale de traction, mesurée à l'aide d'un extensomètre, nécessaire pour décoller les surfaces de 38 mm$^2$, respectives de deux supports (A) et (B), rigides, inertes, non-absorbants, placés en regard l'un de l'autre ; lesdites surfaces étant enduites préalablement par le polymère collant préalablement dissous à 5% dans un solvant aqueux, hydroalcoolique ou alcoolique, à raison de 1 mg/mm$^2$, séchées pendant 24 heures à 22°C sous une humidité relative de 50%, puis soumises pendant 20 secondes à une compression de 3 Newton et enfin soumises pendant 30 secondes à une traction de vitesse 20 mm/minute.

Avantageusement, on utilise des supports (A) et (B) constitués de polyéthylène, de polypropylène, d'alliage métallique ou de verre.

L'énergie de séparation $E_{s(M/V)}$ est l'énergie fournie par l'extensomètre pour effectuer la séparation des surfaces respectives de 38 mm$^2$, de deux supports (C) et (D), rigides, inertes, non absorbants et placés en regard l'un de l'autre ; l'un desdits supports étant constitué de verre poli et l'autre desdits supports étant de nature identique à celle des supports (A) et (B) tels que définis dans la revendication 2 ou 3 et dont la surface est enduite préalablement par le polymère collant préalablement dissous à 5% dans un solvant aqueux, hydroalcoolique ou alcoolique, à raison de 1 mg/mm$^2$, séchées pendant 24 heures à 22°C sous une humidité relative de 50%, les deux surfaces desdits supports (C) et (D) étant soumises pendant 20 secondes à une compression de 3 Newton et enfin soumises pendant 30 secondes à une traction de vitesse 20 mm/minute.

L'énergie de séparation $E_{s(M/V)}$ est un travail qui peut être calculé au moyen de la formule suivante :

$$\int_{X_{s1}+0,05}^{X_{s2}} F(x)\,dx$$

où F(x) est la force nécessaire pour produire un déplacement (x) ;

$x_{s1}$ est le déplacement (exprimé en millimètres) produit par la force de traction maximale ;

$X_{S2}$ où le déplacement (exprimé en millimètres) produit par la force de traction permettant la séparation totale des deux surfaces des supports (C) et (D).

De préférence, on choisira un polymère collant tel que la force maximale de décollement $F_{max}$ soit supérieure à 5 Newton et/ou tel que sa température de transition vitreuse (Tg) soit inférieure à 20°C. Si la Tg du polymère est inférieure à - 15°C, il devra préférentiellement avoir en plus une énergie de séparation $E_{s(M/V)}$ inférieure à 300 µJ.

La concentration relative en poids en polymère collant dans la composition est en général supérieure à 0,01 %, plus préférentiellement supérieure à 0,1%, et plus préférentiellement encore supérieure à 0,5%.

Avantageusement, on choisit un polymère fixant qui présente une température de transition vitreuse (Tg) supérieure à 25°C.

Conformément à l'invention, la concentration relative en poids en polymère fixant dans la composition est en général supérieure à 0,01 %, et de préférence supérieure à 0,1 %.

Une forme particulièrement préférée du polyester sulfonique ramifié est celle obtenue par polymérisation de:

(i) au moins un acide dicarboxylique difonctionnel ne portant pas de fonction sulfonique;

(ii) au moins un monomère difonctionnel portant au moins une fonction sulfonique, le ou les groupements fonctionnels étant choisis dans le groupe comprenant les groupements hydroxyle, carboxyle et amino;

(iii) au moins un diol ou un mélange de diol (s) et de diamine (s);

(iv) éventuellement un monomère difonctionnel choisi dans le groupe comprenant les hydroxyacides carboxyliques, les aminoacides carboxyliques et leurs mélanges;

(v) au moins un réactif multifonctionnel portant au moins trois groupements fonctionnels choisis dans le groupe comprenant les groupements amino, alcool et acide carboxylique.

Une telle polymérisation peut être effectuée à partir de:

(i) au moins un acide dicarboxylique difonctionnel ne portant pas de fonction sulfonique;

(ii) 2 à 15 % relatif en mole de monomère difonctionnel portant au moins une fonction sulfonique;

(iii) au moins un diol ou un mélange de diol (s) et de diamine (s);

(iv) 0 à 40 % relatif en mole du monomère difonctionnel choisi dans le groupe comprenant les hydroxyacides carboxyliques, les aminoacides carboxyliques et leurs mélanges;

(v) 0,1 à 40 % relatif en mole du réactif multifonctionnel portant au moins trois groupements fonctionnels réactifs.

Les polymères sulfoniques ramifiés contiennent de préférence des proportions substantiellement égales, en nombre d'équivalents, d'une part, en fonctions acide carboxylique et d'autre part, en fonctions diol et/ou diol et diamine.

L'acide dicarboxylique difonctionnel (i) est de préférence choisi dans le groupe comprenant les acides aliphatiques dicarboxyliques, les acides alicycliques dicarboxyliques, des acides aromatiques dicarboxyliques ou un mélange de ceux-ci et plus particulièrement dans le groupe comprenant l'acide 1,4 - cyclohexanedioïque, l'acide succinique, l'acide glutarique, l'acide adipique, l'acide azelaïque, acide sebacique, l'acide fumarique, l'acide maléïque, l'acide 1,3 - cyclohexanedioïque, l'acide phtalique, l'acide téréphtaltique et l'acide isophtalique ou leurs mélanges.

Le monomère difonctionnel (ii) tel que défini ci-dessus est de préférence choisi dans le groupe comprenant les acides dicarboxyliques, les esters d'acide dicarboxylique, les glycols et les hydroxyacides contenant, chacun, au moins un groupement métal sulfonate.

Le diol (iii) est de préférence choisi dans le groupe comprenant les alcanediols et les polyalkylènediols et plus particulièrement dans le groupe comprenant l'éthylène glycol, le propylène glycol, le diéthylène glycol, le triéthylène glycol et le polyproprylène glycol.

La diamine (iii) peut être choisie dans le groupe comprenant les alcanediamines et les polyalkylènediamines.

Le réactif multifonctionnel (v) est choisi de préférence dans le groupe comprenant le triméthyloléthane, le triméthylolpropane, le glycérol, le pentaérythritol, le sorbitol, l'anhydride trimellitique, l'érythritol, le threitol, le dipentaérythritol, le dianhydride pyromellitique et l'acide diméthylpropionique.

Les polymères sulfoniques ramifiés particulièrement visés par la présente invention sont ceux décrits dans les demandes de brevets WO 95/181 91, WO 97/082 61 et WO 97/208 99.

Conformément à l'invention, on choisit avantageusement, comme polymère sulfonique ramifié, le polymère AQ 1350 commercialisé par la Société Eastman. Ce polymère AQ 1350 est défini par:

- une température de transition vitreuse donnée par le fournisseur égale à 0°C;
- une force maximale de décollement Fmax égale à 25N.

Le polymère fixant est généralement choisi parmi les polymères fixants anionique, cationique, amphotère, non ionique et leurs mélanges.

Ces polymères fixants peuvent être utilisés sous forme solubilisée ou encore sous forme de dispersion de particules solides de polymère.

En tant que polymère fixant cationique, on choisit préférentiellement les polymères comportant des groupements amine primaire, secondaire, tertiaire et/ou quaternaire faisant partie de la chaîne polymère ou directement reliés à celle-ci, et ayant un poids moléculaire compris entre 500 et environ 5.000.000 et de préférence entre 1000 et 3.000.000.

En tant que polymères fixants anioniques, on préfère les polymères comportant des groupements dérivés d'acide carboxylique, sulfonique ou phosphorique et ont un poids moléculaire moyen en poids compris entre environ 500 et 5.000.000.

En tant que polymères fixants amphotères, on choisit préférentiellement les polymères comportant des motifs B et C répartis statistiquement dans la chaîne polymère, où B désigne un motif dérivant d'un monomère comportant au moins une fonction basique, en particulier un atome d'azote basique et C désigne un motif dérivant d'un monomère acide comportant un ou plusieurs groupements carboxyliques ou sulfoniques ou bien B et C peuvent désigner des groupements dérivant de monomères zwittérioniques de carboxybétaïnes ou de sulfobétaïnes; B et C peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné; ou bien B et C font partie d'une chaîne d'un polymère à motif éthylène $\alpha,\beta$-dicarboxylique dont l'un des groupements carboxyliques a été amené à réagir avec une polyamine comportant un ou plusieurs groupements amine primaire ou secondaire.

En tant que polymères fixants non ioniques, on choisit avantageusement les polyuréthannes.

Parmi les polymères fixants utilisés sous forme solubilisée, on utilisera de préférence les polymères choisis dans le groupe comprenant les polymères acryliques siliconés, les polymères à base de monomère vinyl pyrrolidone et vinyl caprolactame.

Parmi les polymères fixants se présentant sous la forme d'une dispersion, on utilisera de préférence ceux comprenant des monomères acryliques ou métacryliques et leurs esters ou encore ceux comprenant des monomères styrène.

La composition peut se présenter sous forme vaporisable, de mousse, de gel ou de lotion et le véhicule cosmétiquement acceptable peut être constitué par un solvant approprié, auquel sont ajoutés des additifs tels que des agents gélifiants ou des agents moussants. En général, le solvant est choisi parmi l'eau, les alcools ou un mélange hydroalcoolique.

Les compositions peuvent contenir, en outre, une quantité appropriée de propulseurs tels que des gaz comprimés ou liquéfiés usuels, de préférence l'air, le gaz carbonique ou l'azote comprimés, ou encore un gaz soluble ou non dans la composition tel que le diméthyl éther, les hydrocarbures fluorés ou non et leurs mélanges.

L'invention a également pour objet un dispositif aérosol constitué par un récipient contenant une composition aérosol constituée par d'une part une phase liquide (ou jus) contenant au moins une composition conforme à l'invention dans un solvant approprié et un propulseur ainsi qu'un moyen de distribution de ladite composition aérosol.

Encore un autre objet de l'invention est un procédé de traitement des fibres kératiniques, en particulier des cheveux, caractérisé en ce qu'on applique sur lesdites fibres la composition conforme à l'invention, avant ou après la mise en forme de la coiffure.

La composition conforme à l'invention est généralement utilisée dans ou pour la fabrication d'une formulation cosmétique de coiffage.

Les exemples ci-après permettent d'illustrer l'invention sans toutefois chercher à en limiter la portée. On utilisera les polymères indiqués ci-après:

| | |
|---|---|
| Amphomer | Copolymère octylacrylamide /acrylate/ butylaminoéthyl/méthacrylate commercialisé par National Starch |
| Polymer LO-21 DRY | Poly diméthyl / méthyl siloxane à groupements propyl thio-3 acrylate de méthyle / méthacrylate de méthyle / acide méthacrylique commercialisé par 3M |
| Luviskol VA64P | Polyvinylpyrrolidone commercialisé par BASF |
| Uramul SC 132 | Latex copolymère acrylique commercialisé par DMS RESINS; Tg = 50°C |
| AQ 1350 | Polyester sulfonique ramifié commercialisé par la Société Eastman |

EXEMPLES:

On compare ci-après des compositions conformes à l'invention comprenant une association d'un polymère sulfonique ramifié et d'un polymère fixant avec des compositions conformes à l'art antérieur contenant soit le polymère sulfonique ramifié seul, soit le polymère fixant seul.

Exemple 1 (comparatif):

On réalise des tests sensoriels pour comparer la performance de compositions conformes à l'invention et de compositions conformes à l'art antérieur. La comparaison porte sur la tenue dans le temps et sous contrainte de la coiffure.

Pour cela, on réalise 3 compositions conformes à l'invention et 4 compositions conformes à l'art antérieur. On applique ces compositions sur des perruques de cheveux naturels. Puis, on évalue la tenue de la forme de la perruque et le retour de la forme des perruques après agitation.

Composition 1 (invention):

| | |
|---|---|
| AQ 1350 | 4 g |
| Amphomer | 2 g |
| Eau | 75 g |
| 2 Amino-2-méthyl-1-propanol qs neutralisation Amphomer | 0,37 g |
| Alcool qs | 100 g |

Composition 2 (invention):

| | |
|---|---|
| AQ 1350 | 4 g |
| Polymère LO-21 DRY préalablement neutralisé à 90 % | 2 g |
| Eau | 75 g |
| Alcool qs | 100 g |

Composition 3 (invention):

| | |
|---|---|
| AQ 1350 | 4 g |
| LUVISKOL VA 64 P | 2 g |
| Eau | 75 g |
| Alcool qs | 100 g |

Composition 4 (art antérieur- polyester sulfonique ramifié seul):

| AQ 1350 | 6 g |
| Eau | 75 g |
| Alcool qs | 100 g |

Composition 5 (art antérieur- polymère fixant seul):

| Amphomer | 6 g |
| Eau | 75 g |
| 2 Amino 2 méthyl 1 propanol qs neutralisation Amphomer | 1,09 g |
| Alcool qs | 100 g |

Composition 6 (art antérieur- polymère fixant seul):

| Polymer LO-21 DRY préalablement neutralisé à 90% | 6 g |
| Eau | 75 g |
| Alcool qs | 100 g |

Composition 7 (art antérieur- polymère fixant seul):

| LUVISKOL VA 64 P | 6 g |
| Eau | 75 g |
| Alcool qs | 100 g |

On introduit chacune des compositions dans un flacon pompe. On pulvérise 3 grammes de chaque composition sur une perruque de cheveux de 20 cm de longueur préalablement shampooinée et essorée. On laisse sécher pendant 4 heures et on retourne la perruque.

On agite la perruque au moyen d'une rotation alternative pendant 2 heures. On compare la forme finale de la chevelure avec la forme qu'elle avait avant agitation et on estime la tenue de la forme. On utilise la notation de 0 à 5:

- 0 traduit une très mauvaise tenue de la forme et une coiffure entièrement affaissée.
- 5 traduit une excellente tenue et une coiffure restée intacte et volumineuse en dépit de l'agitation.

On démêle ensuite les perruques et on les secoue à nouveau pendant 20 secondes. On estime le retour de la forme de la coiffure lorsqu'elle a subi toutes ces opérations. On utilise la même grille de notation allant de 0 à 5.

Le tableau 1 résume les résultats.

Tableau 1

| Composition | Tenue de la forme après agitation | Retour de la forme après agitation et démêlage |
|---|---|---|
| 1 | 3,25 | 2,5 |
| 2 | 4,0 | 4,0 |
| 3 | 4,5 | 4,25 |
| 4 | 2,0 | 4,0 |

(suite)

| Composition | Tenue de la forme après agitation | Retour de la forme après agitation et démêlage |
|---|---|---|
| 5 | 3,75 | 0,75 |
| 6 | 3,5 | 1,0 |
| 7 | 2,0 | 0,75 |
| sans traitement | 0 | 0,5 |

Le tableau 1 montre que les compositions conformes à l'invention et comprenant l'association de polymères procurent de meilleurs résultats en terme de tenue de la forme après agitation et de retour de la forme après agitation et démêlage que les compositions conformes à l'art antérieur.

Exemple 2:

On réalise une composition 8 conforme à l'invention et on estime la tenue de la coiffure ainsi que certaines propriétés cosmétiques.

Composition 8 (invention):

|  |  |
|---|---|
| AQ 1350 | 4g |
| URAMUL SC 132 | 0,5 g |
| Eau qs | 100 g |

On prend une perruque de 20 g de cheveux naturels, on applique 2,5 grammes de la composition 8 sur les cheveux et on laisse sécher.
On observe que les cheveux présentent un très bon maintien. Le démêlage est facile et la chevelure présente un bon toucher après démêlage.

**Revendications**

1.   Composition cosmétique pour les fibres kératiniques telles que les cheveux, **caractérisée par le fait qu'**elle comprend, dans un milieu cosmétiquement acceptable, au moins un polymère collant de température de transition vitreuse (Tg) inférieure à 20°C et au moins un polymère fixant de température de transition vitreuse (Tg) supérieure à 15°C, le polymère collant étant un polyester sulfonique ramifié.

2.   Composition selon la revendication 1, **caractérisée par le fait que** le polymère collant présente un profil de décollement défini par au moins une force maximale de décollement $F_{max}$ > 3 Newton, et de préférence supérieure à 5N.

3.   Composition selon la revendication 2, **caractérisée par le fait que**, lorsque la température de transition vitreuse du polymère collant est inférieure à -15°C, le profil de décollement est en outre défini par une énergie de séparation $E_{s(M/V)}$ du matériau mis en contact avec une surface en verre, inférieure à 300 µJ.

4.   Composition selon la revendication 2, **caractérisée par le fait que** Fmax est la force maximale de traction, mesurée à l'aide d'un extensomètre, nécessaire pour décoller les surfaces de 38 mm$^2$, respectives de deux supports (A) et (B), rigides, inertes, non absorbants, placés en regard l'un de l'autre ; lesdites surfaces étant enduites préalablement par le polymère collant préalablement dissous à 5% dans un solvant aqueux, hydroalcoolique ou alcoolique, à raison de 1 mg/mm$^2$, séchées pendant 24 heures à 22°C sous une humidité relative de 50 %, puis soumises pendant 20 secondes à une compression de 3 Newton et enfin soumises pendant 30 secondes à une traction de vitesse 20 mm/minute.

5.   Composition selon la revendication 4, **caractérisée par le fait que** les supports (A) et (B) sont constitués de polyéthylène, de polypropylène, d'alliage métallique ou de verre.

**6.** Composition selon la revendication 2, **caractérisée par le fait que** $E_{s(M/V)}$ est l'énergie fournie par l'extensomètre pour effectuer la séparation des surfaces respectives de 38 mm$^2$, de deux supports (C) et (D), rigides, inertes, non absorbants et placés en regard l'un de l'autre ; l'un desdits supports étant constitué de verre poli et l'autre desdits supports étant de nature identique à celle des supports (A) et (B) tels que définis dans la revendication 4 ou 5 et dont la surface est enduite préalablement par le polymère collant préalablement dissous à 5% dans un solvant aqueux, hydroalcoolique ou alcoolique, à raison de 1 mg/mm$^2$, séchées pendant 24 heures à 22°C sous une humidité relative de 50%, les deux surfaces desdits supports (C) et (D) étant soumises pendant 20 secondes à une compression de 3 Newton et enfin soumises pendant 30 secondes à une traction de vitesse 20 mm/minute.

**7.** Composition selon la revendication 6, **caractérisée par le fait que** $E_{s(M/V)}$ est le travail calculé au moyen de la formule suivante :

$$\int_{X_{s1} + 0,05}^{X_{s2}} F(x)dx$$

où F(x) est la force nécessaire pour produire un déplacement (x) ;
$X_{s1}$ est le déplacement (exprimé en millimètres) produit par la force de traction maximale ;
$X_{S2}$ où le déplacement (exprimé en millimètres) produit par la force de traction permettant la séparation totale des deux surfaces des supports (C) et (D).

**8.** Composition selon l'une quelconque des revendications précédentes, **caractérisée par le fait que** la concentration relative en poids en polymère collant dans la composition est supérieure à 0,01 %, de préférence supérieure à 0,1%, et plus préférentiellement encore supérieure à 0,5 %.

**9.** Composition selon l'une quelconque des revendications précédentes, **caractérisée par le fait que** le polymère fixant présente une température de transition vitreuse (Tg) supérieure à 25°C.

**10.** Composition selon l'une quelconque des revendications précédentes, **caractérisée par le fait que** la concentration relative en poids en polymère fixant dans la composition est supérieure à 0,01 %, et de préférence supérieure à 0,1 %.

**11.** Composition selon la revendication 1, **caractérisée par le fait que** le polyester sulfonique ramifié est formé par polymérisation de:

(i) au moins un acide dicarboxylique difonctionnel ne portant pas de fonction sulfonique;
(ii) au moins un monomère difonctionnel portant au moins une fonction sulfonique, le ou les groupements fonctionnels étant choisis dans le groupe comprenant les groupements hydroxyle, carboxyle et amino;
(iii) au moins un diol ou un mélange de diol (s) et de diamine (s);
(iv) éventuellement un monomère difonctionnel choisi dans le groupe comprenant les hydroxyacides carboxyliques, les aminoacides carboxyliques et leurs mélanges;
(v) au moins un réactif multifonctionnel portant au moins trois groupements fonctionnels choisis dans le groupe comprenant les groupements amino, alcool et acide carboxylique.

**12.** Composition selon la revendication 11, **caractérisée par le fait que** la polymérisation est effectuée à partir de:

(i) au moins un acide dicarboxylique difonctionnel ne portant pas de fonction sulfonique;
(ii) 2 à 15 % relatif en mole de monomère difonctionnel portant au moins une fonction sulfonique;
(iii) au moins un diol ou un mélange de diol (s) et de diamine (s);
(iv) 0 à 40 % relatif en mole du monomère difonctionnel choisi dans le groupe comprenant les hydroxyacides carboxyliques, les aminoacides carboxyliques et leurs mélanges;
(v) 0,1 à 40 % relatif en mole du réactif multifonctionnel portant au moins trois groupements fonctionnels réactifs.

**13.** Composition selon l'une quelconque des revendications 11 ou 12, **caractérisée par le fait que** le polymère sulfonique ramifié contient des proportions substantiellement égales, en nombre d'équivalents, d'une part de fonctions acide carboxylique et d'autre part de fonctions diol et/ou diol et diamine.

**14.** Composition selon l'une quelconque des revendications 11 à 13, **caractérisée par le fait que** l'acide dicarboxylique difonctionnel (i) est choisi dans le groupe comprenant les acides aliphatiques dicarboxyliques, les acides alicycliques dicarboxyliques, des acides aromatiques dicarboxyliques.

**15.** Composition selon la revendication 14, **caractérisée par le fait que** l'acide dicarboxylique difonctionnel (i) est choisi dans le groupe comprenant l'acide 1,4 - cyclohexanedioïque, l'acide succinique, l'acide glutarique, l'acide adipique, l'acide azelaïque, acide sebacique, l'acide fumarique, l'acide maléïque, l'acide 1,3 - cyclohexanedioïque, l'acide phtalique, l'acide téréphtaltique et l'acide isophtalique et un mélange de ceux-ci.

**16.** Composition selon l'une quelconque des revendications 11 à 13, **caractérisée par le fait que** le monomère difonctionnel (ii) est choisi dans le groupe comprenant les acides dicarboxyliques, les esters d'acide dicarboxylique, les glycols et les hydroxyacides contenant, chacun, au moins un groupement métal sulfonate.

**17.** Composition selon l'une quelconque des revendications 11 à 13, **caractérisée par le fait que** le diol (iii) est choisi dans le groupe comprenant les alcanediols et les polyalkylènediols.

**18.** Composition selon la revendication 17, **caractérisée par le fait que** le diol (iii) est choisi dans le groupe comprenant l'éthylène glycol, le propylène glycol, le diéthylène glycol, le triéthylène glycol et le polyproprylène glycol.

**19.** Composition selon l'une quelconque des revendications 11 à 13, **caractérisée par le fait que** la diamine (iii) est choisie dans le groupe comprenant les alcanediamines et les polyalkylènediamines.

**20.** Composition selon l'une quelconque des revendications 11 à 13, **caractérisée par le fait que** le réactif multifonctionnel (v) est choisi dans le groupe comprenant le triméthyloléthane, le triméthylolpropane, le glycérol, le pentaérythritol, le sorbitol, l'anhydride trimellitique, l'érythritol, le threitol, le dipentaérythritol, le dianhydride pyromellitique et l'acide diméthylpropionique.

**21.** Composition selon l'une quelconque des revendications précédentes, **caractérisée en ce que** le polymère fixant est choisi parmi les polymères fixants anionique, cationique, amphotère, non ionique et leurs mélanges.

**22.** Composition selon la revendication 21, **caractérisée par le fait que** les polymères fixants se présentent sous forme solubilisée ou sous forme de dispersion de particules solides de polymère.

**23.** Composition selon la revendication 22, **caractérisée en ce que** les polymères fixants cationiques sont choisis parmi les polymères comportant des groupements amine primaire, secondaire, tertiaire et/ou quaternaire faisant partie de la chaîne polymère ou directement reliés à celle-ci, et ayant un poids moléculaire compris entre 500 et environ 5.000.000 et de préférence entre 1000 et 3.000.000.

**24.** Composition selon la revendication 21, **caractérisée en ce que** les polymères fixants anioniques sont des polymères comportant des groupements dérivés d'acide carboxylique, sulfonique ou phosphorique et ont un poids moléculaire moyen en poids compris entre environ 500 et 5.000.000.

**25.** Composition selon la revendication 21, **caractérisée par le fait que** les polymères fixants sont des polymères amphotères choisis parmi les polymères comportant des motifs B et C répartis statistiquement dans la chaîne polymère, où B désigne un motif dérivant d'un monomère comportant au moins une fonction basique, en particulier un atome d'azote basique et C désigne un motif dérivant d'un monomère acide comportant un ou plusieurs groupements carboxyliques ou sulfoniques ou bien B et C peuvent désigner des groupements dérivant de monomères zwittérioniques de carboxybétaïnes ou de sulfobétaïnes; B et C peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné; ou bien B et C font partie d'une chaîne d'un polymère à motif éthylène $\alpha,\beta$-dicarboxylique dont l'un des groupements carboxyliques a été amené à réagir avec une polyamine comportant un ou plusieurs groupements amine primaire ou secondaire.

**26.** Composition selon la revendication 21, **caractérisée en ce que** les polymères fixants non ioniques sont des polyuréthannes.

**27.** Composition selon la revendication 1, **caractérisée par le fait que** le polymère fixant est un polymère hydrosoluble choisi dans le groupe comprenant les polymères acryliques siliconés, les polymères à base de monomère vinyl pyrrolidone et vinyl caprolactame.

28. Composition selon la revendication 1, **caractérisée par le fait que** le polymère fixant est un polymère dispersé à base de monomères acryliques ou méthacryliques et leurs esters et un polymères à base de monomères styrène.

29. Composition selon l'une quelconque des revendications précédentes, **caractérisée en ce qu'**elle se présente sous la forme d'une composition vaporisable, de mousse, de gel ou de lotion.

30. Composition selon l'une quelconque des revendications précédentes, **caractérisée en ce que** le véhicule cosmétiquement acceptable est constitué par un solvant approprié, auquel peuvent être ajoutés des additifs tels que des agents gélifiants ou des agents moussants.

31. Composition selon l'une quelconque des revendications précédentes, **caractérisée en ce qu'**elle comprend un solvant choisi parmi l'eau, un alcool ou un mélange hydroalcoolique.

32. Composition selon l'une quelconque des revendications précédentes, **caractérisée en ce qu'**elle comprend en outre une quantité appropriée de propulseur constitué par les gaz comprimés ou liquéfiés usuels, de préférence l'air, le gaz carbonique ou l'azote comprimés, ou encore un gaz soluble ou non dans la composition tel que le diméthyl éther, les hydrocarbures fluorés ou non, et leurs mélanges.

33. Dispositif aérosol constitué par un récipient contenant une composition aérosol constituée par d'une part une phase liquide (ou jus) contenant au moins une composition selon l'une quelconque des revendications 1 à 28 dans un solvant approprié et un propulseur ainsi qu'un moyen de distribution de ladite composition aérosol.

34. Procédé de traitement des fibres kératiniques, en particulier des cheveux, **caractérisé en ce qu'**on applique sur lesdites fibres la composition telle que définie dans les revendications 1 à 28, avant ou après la mise en forme de la coiffure.

35. Utilisation d'une composition selon l'une quelconque des revendications 1 à 28 dans ou pour la fabrication d'une formulation cosmétique de coiffage.

**Claims**

1. Cosmetic composition for keratinous fibres such as the hair, **characterized in that** it comprises, in a cosmetically acceptable medium, at least one tacky polymer having a glass transition temperature (Tg) of less than 20°C and at least one fixing polymer having a glass transition temperature (Tg) greater than 15°C, the tacky polymer being a branched sulphonic polyester.

2. Composition according to Claim 1, **characterized in that** the tacky polymer has a peeling profile defined by at least one maximum peeling force $F_{max}$ > 3 Newton, and preferably greater than 5 N.

3. Composition according to Claim 2, **characterized in that** when the glass transition temperature of the tacky polymer is less than -15°C, the peeling profile is defined, in addition, by an energy for separation $E_{s(M/V)}$ of the material brought into contact with a glass surface of less than 300 μJ.

4. Composition according to Claim 2, **characterized in that** $F_{max}$ is the maximum tensile force, measured with the aid of an extensometer, necessary to peel apart the respective 38 mm$^2$ surfaces of two rigid, inert and nonabsorbent supports (A) and (B) placed opposite each other; the said surfaces being previously coated with the tacky polymer previously dissolved at 5% in an aqueous, aqueous-alcoholic or alcoholic solvent, at the rate of 1 mg/mm$^2$, dried for 24 hours at 22°C under a relative humidity of 50%, then subjected for 20 seconds to a compression of 3 Newton and finally subjected for 30 seconds to pulling at the rate of 20 mm/min.

5. Composition according to Claim 4, **characterized in that** the supports (A) and (B) consist of polyethylene, polypropylene, metal alloy or glass.

6. Composition according to Claim 2, **characterized in that** $E_{s(M/V)}$ is the energy provided by the extensometer in order to carry out the separation of the respective 38 mm$^2$ surfaces of two rigid, inert and nonabsorbent supports (C) and (D) placed opposite each other; one of the said supports consisting of cut glass and the other of the said supports being of an identical nature to the supports (A) and (B) as defined in Claim 4 or 5 and whose surface is previously coated with the tacky polymer previously dissolved at 5% in an aqueous, aqueous-alcoholic or alcoholic solvent, at the rate of 1 mg/mm$^2$, dried for 24 hours at 22°C under a relative humidity of 50%, the two surfaces of the said supports (C) and (D) being subjected for 20 seconds to a compression of 3 Newton and finally subjected for 30 seconds to pulling at the rate of 20 mm/min.

7.  Composition according to Claim 6, **characterized in that** $E_{s(M/V)}$ is the work calculated by means of the following formula:

$$\int_{x_{s1}+0.05}^{x_{s2}} F(x)dx$$

where F(x) is the force necessary to produce a movement (x);

$x_{s1}$ is the movement (expressed in millimetres) produced by the maximum tensile force;

$x_{s2}$ is the movement (expressed in millimetres) produced by the tensile force which allows the complete separation of the two surfaces of the supports (C) and (D).

8.  Composition according to any one of the preceding claims, **characterized in that** the relative concentration by weight of tacky polymer in the composition is greater than 0.01%, preferably greater than 0.1%, and more preferably still greater than 0.5%.

9.  Composition according to any one of the preceding claims, **characterized in that** the fixing polymer has a glass transition temperature (Tg) greater than 25°C.

10. Composition according to any one of the preceding claims, **characterized in that** the relative concentration by weight of fixing polymer in the composition is greater than 0.01%, and preferably greater than 0.1%.

11. Composition according to Claim 1, **characterized in that** the branched sulphonic polyester is formed by polymerization of:

   (i) at least one difunctional dicarboxylic acid not carrying a sulphonic function;
   (ii) at least one difunctional monomer carrying at least one sulphonic function, the functional group(s) being chosen from the group comprising hydroxyl, carboxyl and amino groups;
   (iii) at least one diol or a mixture of diol(s) and of diamine(s);
   (iv) optionally one difunctional monomer chosen from the group comprising hydroxycarboxylic acids, aminocarboxylic acids and mixtures thereof;
   (v) at least one multifunctional reagent carrying at least three functional groups chosen from the group comprising amino, alcohol and carboxylic acid groups.

12. Composition according to Claim 11, **characterized in that** the polymerization is carried out starting with:

   (i) at least one difunctional dicarboxylic acid not carrying a sulphonic function;
   (ii) 2 to 15 relative mol% of difunctional monomer carrying at least one sulphonic function;
   (iii) at least one diol or a mixture of diol(s) and of diamine(s);
   (iv) 0 to 40 relative mol% of the difunctional monomer chosen from the group comprising the hydroxycarboxylic acids, the aminocarboxylic acids and mixtures thereof;
   (v) 0.1 to 40 relative mol% of the multifunctional reagent carrying at least three reactive functional groups.

13. Composition according to either of Claims 11 and 12, **characterized in that** the branched sulphonic polymer contains substantially equal proportions, as number of equivalents, of carboxylic acid functions, on the one hand, and of diol and/or diol and diamine functions, on the other hand.

14. Composition according to any one of Claims 11 to 13, **characterized in that** the difunctional dicarboxylic acid (i) is chosen from the group comprising aliphatic dicarboxylic acids, alicyclic dicarboxylic acids and aromatic dicarboxylic acids.

15. Composition according to Claim 14, **characterized in that** the difunctional dicarboxylic acid (i) is chosen from the group comprising 1,4-cyclohexanedioic acid, succinic acid, glutaric acid, adipic acid, azelaic acid, sebacic acid, fumaric acid, maleic acid, 1,3-cyclohexanedioic acid, phthalic acid, terephthalic acid and isophthalic acid and a mixture thereof.

16. Composition according to any one of Claims 11 to 13, **characterized in that** the difunctional monomer (ii) is chosen from the group comprising dicarboxylic acids, dicarboxylic acid esters, glycols and hydroxy acids each containing at least one metal sulphonate group.

17. Composition according to any one of Claims 11 to 13, **characterized in that** the diol (iii) is chosen from the group comprising alkane diols and polyalkylene diols.

18. Composition according to Claim 17, **characterized in that** the diol (iii) is chosen from the group comprising ethylene glycol, propylene glycol, diethylene glycol, triethylene glycol and polypropylene glycol.

19. Composition according to any one of Claims 11 to 13, **characterized in that** the diamine (iii) is chosen from the group comprising alkanediamines and polyalkylenediamines.

20. Composition according to any one of Claims 11 to 13, **characterized in that** the multifunctional reagent (v) is chosen from the group comprising trimethylolethane, trimethylolpropane, glycerol, pentaerythritol, sorbitol, trimellitic anhydride, erythritol, threitol, dipentaerythritol, pyromellitic dianhydride and dimethylpropionic acid.

21. Composition according to any one of the preceding claims, **characterized in that** the fixing polymer is chosen from the anionic, cationic, amphoteric and nonionic fixing polymers and mixtures thereof.

22. Composition according to Claim 21, **characterized in that** the fixing polymers are provided in solubilized form or in the form of a dispersion of solid particles of polymer.

23. Composition according to Claim 22, **characterized in that** the cationic fixing polymers are chosen from the polymers comprising primary, secondary, tertiary and/or quaternary amine groups which are part of the polymer chain or which are directly attached to it, and having a molecular weight of between 500 and about 5,000,000 and preferably between 1000 and 3,000,000.

24. Composition according to Claim 21, **characterized in that** the anionic fixing polymers are polymers comprising groups derived from a carboxylic, sulphonic or phosphoric acid and which have a weight-average molecular weight of between about 500 and 5, 000, 000.

25. Composition according to Claim 21, **characterized in that** the fixing polymers are amphoteric polymers chosen from the polymers comprising B and C units randomly distributed in the polymer chain, where B denotes a unit derived from a monomer comprising at least one basic function, in particular a basic nitrogen atom and C denotes a unit derived from an acidic monomer comprising one or more carboxylic or sulphonic groups or alternatively B and C may denote groups derived from zwitterionic monomers of carboxybetaines or sulphobetaines; B and C may also denote a cationic polymer chain comprising primary, secondary, tertiary or quaternary amine groups, in which at least one of the amine groups carries a carboxylic or sulphonic group attached via a hydrocarbon radical; or alternatively B and C are part of a chain of a polymer containing an ethylene-$\alpha,\beta$-dicarboxylic unit of which one of the carboxylic groups has been caused to react with a polyamine comprising one or more primary or secondary amine groups.

26. Composition according to Claim 21, **characterized in that** the nonionic fixing polymers are polyurethanes.

27. Composition according to Claim 1, **characterized in that** the fixing polymer is a watersoluble polymer chosen from the group comprising silicone-containing acrylic polymers, polymers based on a vinylpyrrolidone and vinylcaprolactam monomer.

28. Composition according to Claim 1, **characterized in that** the fixing polymer is a dispersed polymer based on acrylic or methacrylic monomers and esters thereof and a polymer based on styrene monomers.

**29.** Composition according to any one of the preceding claims, **characterized in that** it is provided in the form of a vaporizable composition, a foam, a gel or a lotion.

**30.** Composition according to any one of the preceding claims, **characterized in that** the cosmetically acceptable vehicle consists of an appropriate solvent, to which additives such as gelling agents or foaming agents may be added.

**31.** Composition according to any one of the preceding claims, **characterized in that** it comprises a solvent chosen from water, an alcohol or an aqueous-alcoholic mixture.

**32.** Composition according to any one of the preceding claims, **characterized in that** it comprises, in addition, an appropriate quantity of propellant consisting of customary compressed or liquefied gases, preferably compressed air, carbon dioxide or nitrogen, or alternatively a gas which is soluble or otherwise in the composition, such as dimethyl ether, hydrocarbons which are fluorinated or otherwise and mixtures thereof.

**33.** Aerosol device consisting of a container containing an aerosol composition consisting, on the one hand, of a liquid phase (or juice) containing at least one composition in accordance with any one of Claims 1 to 28 in an appropriate solvent and a propellant as well as a means of distributing the said aerosol composition.

**34.** Method of treating keratinous fibres, in particular hair, **characterized in that** the composition as defined in Claims 1 to 28 is applied to the said fibres before or after shaping the hairstyle.

**35.** Use of a composition according to any one of Claims 1 to 28 in or for making a cosmetic hairstyling formulation.

**Patentansprüche**

**1.** Kosmetische Zusammensetzung für Keratinfasern, wie Haare, **dadurch gekennzeichnet, dass** sie in einem kosmetisch akzeptablen Medium mindestens ein klebriges Polymer mit einer Glasübergangstemperatur (Tg) unter 20 °C und mindestens ein fixierendes Polymer mit einer Glasübergangstemperatur (Tg) über 15 °C aufweist, wobei es sich bei dem klebrigen Polymer um einen verzweigten Sulfonsäurepolyester handelt.

**2.** Zusammensetzung nach Anspruch 1, **dadurch gekennzeichnet, dass** das klebrige Polymer ein Ablösungsprofil aufweist, das zumindest durch eine maximale Ablösungskraft $F_{max}$ > 3 Newton und vorzugsweise größer 5N definiert ist.

**3.** Zusammensetzung nach Anspruch 2, **dadurch gekennzeichnet, dass**, wenn die Glasübergangstemperatur des klebrigen Polymers unter -15 °C liegt, das Ablösungsprofil ferner durch eine Trennenergie $E_{s(M/V)}$ des Materials in Kontakt mit einer Glasfläche unter 300 μJ definiert ist.

**4.** Zusammensetzung nach Anspruch 2, **dadurch gekennzeichnet, dass** $F_{max}$ die mit einem Extensometer gemessene maximale Zugkraft ist, die erforderlich ist, um die Oberflächen von zwei Trägern (A) und (B) von 38 mm$^2$, die steif, inert und nicht absorbierend und aneinander gebracht sind, abzulösen, wobei die Oberflächen zuvor mit dem vorab in einem wässerigen, wässerig-alkoholischen oder alkoholischen Lösungsmittel in einer Menge von 5 % gelöstem klebrigen Polymer in einer Menge von 1 mg/mm$^2$ beschichtet, 24 Stunden bei 22 °C in einer relativen Luftfeuchte von 50 % getrocknet und anschließend 20 Sekunden mit 3 Newton zusammengepresst und schließlich 30 Sekunden einer Zuggeschwindigkeit von 20 mm/Minute ausgesetzt werden.

**5.** Zusammensetzung nach Anspruch 4, **dadurch gekennzeichnet, dass** die Träger (A) und (B) aus Polyethylen, Polypropylen, einer Metalllegierung oder Glas bestehen.

**6.** Zusammensetzung nach Anspruch 2, **dadurch gekennzeichnet, dass** $E_{s(M/V)}$ die Energie ist, die von dem Extensometer aufgebracht werden muss, um Oberflächen von zwei starren, inerten nichtabsorbierenden und aneinander angebrachten Trägern (C) und (D) von 38 mm$^2$ zu trennen, wobei ein Träger aus poliertem Glas besteht und der andere Träger identisch ist mit den Trägern (A) und (B), wie sie in Anspruch 4 oder 5 definiert wurden, und dessen Oberfläche vorab mit einem klebrigen Polymer, das zuvor in einer Menge von 5 % in einem wässerigen, wässerig-alkoholischen oder alkoholischen Lösungsmittel gelöst wurde, in einer Menge von 1 mg/mm$^2$ beschichtet und 24 Stunden bei 22 °C bei einer relativen Luftfeuchte von 50 % getrocknet wird, wobei die beiden Träger (C) und (D) 20 Sekunden einer Kompression von 3 Newton und anschließend 30 Sekunden einer Zuggeschwindigkeit von 20

**13**

mm/Minute ausgesetzt werden.

7. Zusammensetzung nach Anspruch 6, **dadurch gekennzeichnet, dass** $E_{s(M/V)}$ die mit der folgenden Formel berechnete Arbeit ist:

$$\int_{x_{s1}+0,05}^{x_{s2}} F(x)\,dx$$

wobei F(x) die Kraft ist, die erforderlich ist, um eine Verschiebung (x) zu erzielen;
$x_{s1}$ ist die Verschiebung (ausgedrückt in Millimetern), die durch die maximale Zugkraft bewirkt wird;
$x_{s2}$ ist die Verschiebung (ausgedrückt in Millimetern), die durch die Zugkraft bewirkt wird, die die vollständige Trennung der beiden Oberflächen der Träger (C) und (D) bewirkt.

8. Zusammensetzung nach einem der vorhergehenden Ansprüche, **dadurch gekennzeichnet, dass** die relative Gewichtskonzentration des klebrigen Polymers in der Zusammensetzung größer als 0,01 %, vorzugsweise größer als 0,1 % und noch bevorzugter größer 0,5 % ist.

9. Zusammensetzung nach einem der vorhergehenden Ansprüche, **dadurch gekennzeichnet, dass** das fixierende Polymer eine Glasübergangstemperatur (Tg) über 25 °C besitzt.

10. Zusammensetzung nach einem der vorhergehenden Ansprüche, **dadurch gekennzeichnet, dass** die relative Konzentration des fixierenden Polymers in der Zusammensetzung größer als 0,01 Gew.-% und vorzugsweise größer 0,1 Gew.-% ist.

11. Zusammensetzung nach Anspruch 1, **dadurch gekennzeichnet, dass** der verzweigte Sulfonsäurepolyester durch Polymerisation der folgenden Verbindungen gebildet wird:

(i) mindestens einer bifunktionellen Dicarbonsäure, die keine Sulfonsäurefunktion trägt;
(ii) mindestens eines bifunktionellen Monomers, das mindestens eine Sulfonsäurefunktion trägt, wobei die funktionelle(n) Gruppe(n) unter den Gruppen ausgewählt sind, die Hydroxy-, Carboxy- und Aminogruppen aufweisen;
(iii) mindestens eines Diols oder eines Gemisches aus Diol(en) und Diamin(en);
(iv) gegebenenfalls eines bifunktionellen Monomers, das unter den Hydroxycarbonsäuren, Aminocarbonsäuren und deren Gemischen ausgewählt ist;
(v) mindestens eines multifunktionellen Reaktanten, der mindestens drei funktionelle Gruppen aufweist, die unter den Gruppen Amino, Alkohol und Carbonsäure ausgewählt sind.

12. Zusammensetzung nach Anspruch 11, **dadurch gekennzeichnet, dass** die Polymerisation ausgehend von den folgenden Verbindungen durchgeführt wird:

(i) mindestens einer bifunktionellen Dicarbonsäure, die keine Sulfonsäurefunktion trägt;
(ii) 2 bis 15 Mol-% eines bifunktionellen Monomers, das mindestens eine Sulfonsäurefunktion aufweist;
(iii) mindestens eines Diols oder eines Gemisches aus Diol(en) und Diamin(en);
(iv) 0 bis 40 Mol-% eines bifunktionellen Monomers, das unter den Hydroxycarbonsäuren, Aminocarbonsäuren und deren Gemischen ausgewählt ist;
(v) 0,1 bis 40 Mol-% eines multifunktionellen Reaktanten, der mindestens drei reaktive funktionelle Gruppen aufweist.

13. Zusammensetzung nach einem der Ansprüche 11 oder 12, **dadurch gekennzeichnet, dass** das verzweigte Sulfonsäurepolymer in in etwa gleichen Mengenanteilen, ausgedrückt als Zahl der Äquivalente, einerseits Carbonsäurefunktionen und andererseits Diolfunktionen und/ oder Diol- und Diaminfunktionen enthält.

14. Zusammensetzung nach einem der Ansprüche 11 bis 13, **dadurch gekennzeichnet, dass** die bifunktionelle Dicarbonsäure (i) unter den aliphatischen Dicarbonsäuren, alicyclischen Dicarbonsäuren und aromatischen Dicarbonsäuren ausgewählt ist.

**15.** Zusammensetzung nach Anspruch 14, **dadurch gekennzeichnet, dass** die bifunktionelle Dicarbonsäure (i) unter 1,4-Cyclohexandisäure, Bernsteinsäure, Glutarsäure, Adipinsäure, Azelainsäure, Sebacinsäure, Fumarsäure, Maleinsäure, 1,3-Cyclohexandisäure, Phthalsäure, Terephthalsäure und Isophthalsäure und deren Gemischen ausgewählt ist.

**16.** Zusammensetzung nach einem der Ansprüche 11 bis 13, **dadurch gekennzeichnet, dass** das bifunktionelle Monomer (ii) unter den Dicarbonsäuren, Dicarbonsäureestern, Glycolen und Hydroxysäuren, die jeweils mindestens eine Metallsulfonatgruppe aufweisen, ausgewählt ist.

**17.** Zusammensetzung nach einem der Ansprüche 11 bis 13, **dadurch gekennzeichnet, dass** das Diol (iii) unter den Alkandiolen und Polyalkylendiolen ausgewählt ist.

**18.** Zusammensetzung nach Anspruch 17, **dadurch gekennzeichnet, dass** das Diol (iü) unter Ethylenglycol, Propylenglycol, Diethylenglycol, Triethylenglycol und Polypropylenglycol ausgewählt ist.

**19.** Zusammensetzung nach einem der Ansprüche 11 bis 13, **dadurch gekennzeichnet, dass** das Diamin (iii) unter den Alkandiaminen und Polyalkylendiaminen ausgewählt ist.

**20.** Zusammensetzung nach einem der Ansprüche 11 bis 13, **dadurch gekennzeichnet, dass** der multifunktionelle Reaktant (v) unter Trimethylolethan, Trimethylolpropan, Glycerin, Pentaerythrit, Sorbit, Trimellitanhydrid, Erythrit, Threit, Dipentaerythrit, Pyromellitdianhydrid und Dimethylpropylpropionsäure ausgewählt ist.

**21.** Zusammensetzung nach einem der vorhergehenden Ansprüche, **dadurch gekennzeichnet, dass** das fixierende Polymer unter den anionischen, kationischen, amphoteren oder nichtionischen fixierenden Polymeren und deren Gemischen ausgewählt ist.

**22.** Zusammensetzung nach Anspruch 21, **dadurch gekennzeichnet, dass** die fixierenden Polymere in solubilisierter Form oder als Dispersion von festen Polymerpartikeln vorliegen.

**23.** Zusammensetzung nach Anspruch 22, **dadurch gekennzeichnet, dass** die kationischen fixierenden Polymere unter den Polymeren ausgewählt sind, die primäre, sekundäre, tertiäre und/oder quartäre Aminogruppen enthalten, die Teil der Polymerkette sind oder direkt daran gebunden sind, und die eine Molmasse von 500 bis etwa 5.000.000 und vorzugsweise 1.000 bis 3.000.000 aufweisen.

**24.** Zusammensetzung nach Anspruch 21, **dadurch gekennzeichnet, dass** die anionischen fixierenden Polymere Polymere sind, die Gruppen aufweisen, die von Carbonsäuren, Sulfonsäuren oder Phosphorsäuren abgeleitet sind und deren gewichtsmittlere Molmasse im Bereich von etwa 500 bis 5.000.000 liegt.

**25.** Zusammensetzung nach Anspruch 21, **dadurch gekennzeichnet, dass** die fixierenden Polymere amphotere Polymere sind, die unter den Polymeren ausgewählt sind, die statistisch in der Polymerkette verteilte Einheiten B und C enthalten, wobei B eine Einheit ist, die von einem Monomer abgeleitet ist, das mindestens eine basische Funktion und insbesondere ein basisches Stickstoffatom enthält, und C eine Einheit bedeutet, die von einem sauren Monomer abgeleitet ist, das eine oder mehrere Carbonsäuregruppen oder Sulfonsäuregruppen enthält, oder B und C Gruppen bedeuten können, die von zwitterionischen Carboxybetain- oder Sulfobetainmonomeren abgeleitet sind; B und C können auch eine kationische Polymerkette bedeuten, die primäre, sekundäre, tertiäre oder quartäre Aminogruppen enthält, bei der mindestens eine Aminogruppe eine Carbonsäure oder Sulfonsäuregruppe trägt, die über eine Kohlenwasserstoffgruppe gebunden ist; oder B und C sind Teil einer Polymerkette mit $\alpha,\beta$-Dicarboxyethyleneinheiten, bei der eine Carboxygruppe mit einem Polyamin umgesetzt wurde, das eine oder mehrere primäre oder sekundäre Aminogruppen aufweist.

**26.** Zusammensetzung nach Anspruch 21, **dadurch gekennzeichnet, dass** die nichtionischen festigenden Polymere Polyurethane sind.

**27.** Zusammensetzung nach Anspruch 1, **dadurch gekennzeichnet, dass** das festigende Polymer ein wasserlösliches Polymer ist, das unter den silicionierten Acrylpolymeren, Polymeren auf der Basis eines Vinylpyrrolidonmonomers und Vinylcaprolactam ausgewählt ist.

**28.** Zusammensetzung nach Anspruch 1, **dadurch gekennzeichnet, dass** das fixierende Polymer ein dispergiertes Polymer auf der Basis von Acryl- oder Methacrylmonomeren und deren Estern und ein Polymer auf der Basis von Styrolmonomeren ist.

29. Zusammensetzung nach einem der vorhergehenden Ansprüche, **dadurch gekennzeichnet, dass** sie als zerstäubbare Zusammensetzung, Schaum, Gel oder Lotion vorliegt.

30. Zusammensetzung nach einem der vorhergehenden Ansprüche, **dadurch gekennzeichnet, dass** der kosmetisch akzeptable Träger aus einem geeigneten Lösungsmittel besteht, dem Zusatzstoffe, wie Gelbildner oder Schaumbildner zugesetzt sein können.

31. Zusammensetzung nach einem der vorhergehenden Ansprüche, **dadurch gekennzeichnet, dass** sie ein Lösungsmittel enthält, das unter Wasser, Alkoholen oder wässerig-alkoholischen Gemischen ausgewählt ist.

32. Zusammensetzung nach einem der vorhergehenden Ansprüche, **dadurch gekennzeichnet, dass** sie ferner eine geeignete Menge eines Treibmittels enthält, das unter den üblichen Druckgasen oder Flüssiggasen und vorzugsweise Luft, Kohlendioxid oder Stickstoff, die komprimiert sind, oder einem in der Zusammensetzung löslichen oder unlöslichen Gas ausgewählt ist, wie Dimethylether, fluorierten oder nichtfluorierten Kohlenwasserstoffen und deren Gemischen.

33. Aerosolvorrichtung, die aus einem Behälter, der eine Aerosolzusammensetzung, die aus einer flüssigen Phase (oder Flüssigkeit) mit mindestens einer Zusammensetzung nach einem der Ansprüche 1 bis 28 in einem geeigneten Lösungsmittel und einem Treibmittel besteht, und einer Einrichtung zur Verteilung der Aerosolzusammensetzung besteht.

34. Verfahren zur Behandlung von Keratinfasern, besonders zur Behandlung der Haare, **dadurch gekennzeichnet, dass** auf die Fasern die Zusammensetzung nach einem der Ansprüche 1 bis 28 aufgebracht wird, bevor oder nachdem die Frisur geformt wurde.

35. Verwendung einer Zusammensetzung nach einem der Ansprüche 1 bis 28 in einer kosmetischen Formulierung für die Frisurengestaltung oder zur Herstellung einer kosmetischen Formulierung für die Frisurengestaltung.